# EUROPEAN PATENT SPECIFICATION

(11) **EP 1 950 197 B1**
(45) Date of publication and mention of the grant of the patent: **30.04.2014**
(21) Application number: 05800404.5
(22) Date of filing: 31.10.2005
(51) Int. Cl.: C08G 18/76, C08G 18/38, G02B 1/04, C07C 319/12, C07C 323/52

(54) **PROCESS FOR PRODUCING PENTAERYTHRITOL MERCAPTOCARBOXYLIC ACID ESTER, PENTAERYTHRITOL MERCAPTOCARBOXYLIC ACID ESTER OBTAINED BY THE SAME, AND USE THEREOF**
VERFAHREN ZUR HERSTELLUNG VON PENTAERYTHRITMERCAPTOCARBONSÄUREESTER, DURCH DIESES ERHALTENER PENTAERYTHRITMERCAPTOCARBONSÄUREESTER UND VERWENDUNG DAVON
PROCEDE DE FABRICATION D UN ESTER DE L ACIDE PENTAERYTHRITOL MERCAPTOCARBOXYLIQUE, ESTER DE L ACIDE PENTAERYTHRITOL MERCAPTOCARBOXYLIQUE OBTENU PAR CE PROCEDE ET SON UTILISATION

(43) Date of publication of application: 30.07.2008
(73) Proprietor: Mitsui Chemicals, Inc., Tokyo 105-7117 (JP)
(72) Inventor: KUMA, Shigetoshi, Omuta-shi, Fukuoka 836-8610 (JP); TOKUNAGA, Koichi, Omuta-shi, Fukuoka 836-8610 (JP); FUKATSU, Norihiko, Omuta-shi, Fukuoka 836-8610 (JP); KOBAYASHI, Seiichi, Omuta-shi, Fukuoka 836-8610 (JP)
(74) Representative: Wills, Andrew Jonathan
(86) International application number: PCT/JP2005/019991
(87) International publication number: WO 2007/052329

(56) References cited:
- GB-A- 799 182
- JP-A- 10 120 646
- JP-A- 2001 329 110
- JP-A- 2003 261 711
- JP-A- 2005 336 104
- 'TCI CATALOG 34' 1998 TOKYO KASEI ORGANIC CHEMICALS 1998, page 1185, XP003007356
- 'aldrich' ALDRICH CHEMICAL COMPAGNY 1990, page 1011, XP003007357

## Description

### TECHNICAL FIELD

The present invention relates to a process for producing a pentaerythritol mercaptocarboxylic acid ester, a pentaerythritol mercaptocarboxylic acid ester obtained by the process, a polymerizable composition containing the pentaerythritol mercaptocarboxylic acid ester, a resin obtained by the polymerizable composition, and an optical element and lens made of the resin.

### BACKGROUND ART

Since a plastic lens is lightweight and hardly broken as compared to an inorganic lens, and can be dyed, in late years, it has quickly come into wide use as an optical element of spectacle lenses, camera lenses and the like.

Further high performance resins for plastic lenses have been required and high refractive index, high Abbe number, low specific gravity, high heat resistance and the like have been in demand. Various resin materials for lenses have hitherto been developed and used accordingly.

Of such materials, polyurethane based resins have been actively proposed. The present inventors have also proposed plastic lenses using the polyurethane based resin in various ways (refer to Patent Documents 1, 2 and 3).

Of such materials, as the most typical resin, a resin obtained by reacting a pentaerythritol mercaptocarboxylic acid ester with a polyiso(thio)cyanate compound is colorless, is transparent, has a high refractive index and is low dispersion. It is one of resins which are the most suitable for plastic lenses excellent in impact properties, dyeing properties, processability and the like.

A pentaerythritol mercaptocarboxylic acid ester has been produced by a so-called direct esterification method which is conducted while removing by-produced water from the system in the presence of an esterification catalyst using a polyhydric alcohol in general and a mercaptocarboxylic acid (refer to Patent Document 4).

Pentaerythritol, a raw material of the ester compound, is usually produced by subjecting acetaldehyde and formaldehyde to condensation. The purity thereof is usually about 90 weight % and a variety of impurities are contained. Among them, bispentaerythritol that is a bimolecular condensate of formaldehyde of pentaerythritol may be cited. When the bispentaerythritol is contained in an amount of exceeding 5 weight %, there have been known problems such that it is difficult to release from the mold after polymerization with a polyisocyanate compound is completed, there are generated bubbles inside the obtained lens, and the like (refer to Patent Documents 5 and 6).

Patent Document 7 describes improvements to the purification of pentaerythritol, in particular to the purification of crude pentaerythritol. The document describes a process which can be utilized for the purification of technical grade pentaerythritol containing a minor amount of bispentaerythritol monoformal to remove all or a substantial amount of the bispentaerythritol monoformal that is contained within. The process requires the use of a strongly acidic cation exchange resin at a specified flow rate and temperature, flowed by crystallisation of the product (without crystallisation of the bispentaerythritol monoformal).
Patent Document 1: Japanese Patent Laid-open No. S60(1985)-199016
Patent Document 2: Japanese Patent Laid-open No. S60(1985)-217229
Patent Document 3: Japanese Patent Laid-open No. S63(1988)-46213
Patent Document 4: Japanese Patent Publication No. S39(1964)-9071
Patent Document 5: Japanese Patent Laid-open No. S56(1981)-20530
Patent Document 6: Japanese Patent Laid-open No. H10(1998)-120646
Patent Documnet 7: GB 799,182

### DISCLOSURE OF THE INVENTION

Such pentaerythritol containing bispentaerythritol is capable to have the content of bispentaerythritol of not more than 5 weight %, for example, by subjecting the pentaerythritol including bispentaerythritol to a heating process at 160 degree centigrade to 200 degree centigrade. Further, there has been known that it can be purified to pentaerythritol having the content of not less than 98 weight % (refer to Patent Document 5). However, even when the thus-purified pentaerythritol is used, a lens obtained by subjecting the obtained pentaerythritol mercaptocarboxylic acid ester and a polyisocyanate compound to polymerization is whitened in some cases so that suppression of whitening has been desired.

In order to solve the foregoing objects, the present inventors have conducted an extensive study and as a result, have decided that whitening of a polyurethane based lens is caused by a pentaerythritol mercaptocarboxylic acid ester that is a monomer. Furthermore, they have continuously conducted an extensive study and as a result, have found that when a pentaerythritol mercaptocarboxylic acid ester prepared by using pentaerythritol having a total amount of sodium and calcium of not more than a specific amount is used as a raw material, remarkably enough, a colorless and transparent polyurethane based lens in which the above problem is solved, that is, whitening is suppressed is obtained. Thus, the present invention has been completed.

Thus the invention relates to a process for producing a pentaerythritol mercaptocarboxylic acid ester including reacting pentaerythritol which contains no more than 0.2% by weight of Na and Ca, in total, and contains not more than 5.0% by weight of bispentaerythritol, with a mercaptocarboxylic acid.

The pentaerythritol mercaptocarboxylic acid ester obtained by the aforementioned production process may be used to provide a polymerizable composition composed of the pentaerythritol mercaptocarboxylic acid ester and a polyiso(thio)cyanate compound. A resin can be obtained by curing the polymerizable composition.

An optical element such as a lens may be composed of the resin.

By means of the present invention, it is possible to obtain a pentaerythritol mercaptocarboxylic acid ester that is not whitened even though it is reacted with poly (iso) thiocyanate. Accordingly, it is possible to provide a colorless and transparent polyurethane based lens excellent in optical properties in which whitening is suppressed.

### BEST MODE FOR CARRYING OUT THE INVENTION

The embodiments of the present invention will be illustrated below.

A process for producing a pentaerythritol mercaptocarboxylic acid ester according to the embodiment of the present invention includes reacting pentaerythritol which contains no more than 0.2% by weight of Ca and Na in total, and no more than 5.0% by weight of bispentaerythritol, with a mercaptocarboxylic acid.

Herein, when the content of bispentaerythritol is not more than 5.0 weight %, it is possible to obtain a polyurethane based lens which is excellent in mold release properties and in which no bubbles are generated. The content of bispentaerythritol can be measured by gas chromatography as described, for example, in Patent Document 5, or can be measured by high performance liquid chromatography as described in Patent Document 6.

The content of bispentaerythritol may also be reduced, for example, by properly adopting a conventionally known method. For example, as described in Patent Document 5, bispentaerythritol may be heated at 160 degree centigrade to 200 degree centigrade, while as described in Patent Document 6, bispentaerythritol may be hydrolyzed while heat-refluxing under a nitrogen atmosphere. By properly applying these means, pentaerythritol with the content of bispentaerythritol of not more than 5.0 weight % may be obtained.

Furthermore, when the total amount of Ca and Na in the pentaerythritol is not more than 0.2 weight %, a polyurethane based lens obtained by subjecting a pentaerythritol mercaptocarboxylic acid ester prepared by using the pentaerythritol and polyiso(thio)cyanate to polymerization becomes a colorless and transparent polyurethane based lens in which whitening is suppressed.

Methods for measuring the content of metal elements are as follows. Light metals such as Na can be quantitatively analyzed by an ion chromatographic method after pentaerythritol becomes an aqueous solution. Heavy metals can be quantitatively analyzed by using an atomic absorption spectrometric method for measuring the absorbance, or a plasma emission spectrometer, after pentaerythritol is incinerated and then dissolved in a color solution for coloring. The content of sodium and calcium, may be reduced by taking a measure to reduce metal components. For example, the metal components can be reduced by an acid treatment using hydrochloric acid or sulfuric acid, and can also be reduced by a recrystallization method using a water system.

The mercaptocarboxylic acid that is the other raw material is a compound having one or more mercapto groups and one or more carboxyl groups in a molecule, and is not particularly limited in terms of the quality. General industrial chemicals are suitably used. Concrete examples thereof include thioglycolic acid, thiolactic acid, 3-mercaptopropionic acid, thiomalic acid, and thiosalicylic acid, but the present invention is not restricted to these exemplified compounds. Furthermore, these compounds may be reacted singly or in combination of two or more compounds with pentaerythritol.

As the esterification catalyst which is usually used to react pentaerythritol with a mercaptocarboxylic acid, for example, preferably used are acid catalysts represented by mineral acids such as sulfuric acid, hydrochloric acid, phosphoric acid and alumina, and organic acids such as p-toluenesulfonic acid, benzenesulfonic acid, methanesulfonic acid, trichloroacetic acid, and dibutyltin oxide.

In the production of the pentaerythritol mercaptocarboxylic acid ester, an azeotropic agent is not necessarily used, but there is generally used a process for continuously removing by-produced water from the system under heating reflux using an azeotropic agent. Examples of the azeotropic agent which is usually used include, for example, benzene, toluene, xylene, nitrobenzene, chlorobenzene, dichlorobenzene, anisole, diphenyl ether, methylene chloride, chloroform and dichloroethane. These may be used in combination of two or more kinds, or after mixed with other solvents.

As the pentaerythritol mercaptocarboxylic acid ester obtained by the aforementioned process of the embodiment, for example, the following compounds may be cited. Examples thereof include pentaerythritol thioglycolic acid ester, pentaerythritol 3-mercaptopropionic acid ester, pentaerythritol thiolactic acid ester and pentaerythritol thiosalicylic acid ester. However, the present invention is not restricted to the exemplified compounds. Furthermore, these ester compounds may be a compound in which a hydroxy group of pentaerythritol is completely esterified or a compound in which only a part thereof is esterified. Furthermore, these ester compounds may be used in combination of two or more kinds.

A polymerizable composition may be obtained from the foregoing pentaerythritol mercaptocarboxylic acid ester and a polyiso(thio)cyanate compound.

The polyiso(thio)cyanate compound used for the polymerizable composition is a compound having at least two or more iso(thio)cyanate groups in a molecule and is not particularly limited. Concrete examples thereof include aliphatic polyisocyanate compounds such as hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1, 6, 11-undecane triisocyanate, 1,3,6-hexamethylene triisocyanate, 1,8-diisocyanato-4-isocyanatomethyloctane, bis(isocyanatoethyl)carbonate, bis(isocyanatoethyl)ether, lysine diisocyanate methyl ester and lysine triisocyanate;
polyisocyanate compounds having aromatic ring compounds such as 1,2-diisocyanatobenzene, 1,3-diisocyanatobenzene, 1,4-diisocyanatobenzene, 2,4-diisocyanatotoluene, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, tolidine diisocyanate, 4,4'-methylenebis(phenyl isocyanate), 4,4'-methylenebis(2-methylphenyl isocyanate), bibenzyl-4,4'-diisocyanate, bis(isocyanatophenyl)ethylene, bis(isocyanatoethyl)benzene, bis(isocyanatopropyl)benzene, α,α,α',α'-tetramethylxylylene diisocyanate, bis(isocyanatobutyl)benzene, bis(isocyanatomethyl)naphthalene, bis(isocyanatomethylphenyl) ether, bis(isocyanatoethyl)phthalate, and 2,6-di (isocyanatomethyl) furan;
sulfur-containing aliphatic polyisocyanate compounds such as bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-thiapentane, 1,2,3-tris(isocyanatomethylthio)propane, 1,2,3-tris(isocyanatoethylthio)propane, 3,5-dithia-1,2,6,7-heptane tetraisocyanate, 2,6-diisocyanatomethyl-3,5-dithia-1,7-heptane diisocyanate, 2,5-diisocyanatomethylthiophene and 4-isocyanatoethylthio-2,6-dithia-1,8-octane diisocyanate;
aromatic sulfide based polyisocyanate compounds such as 2-isocyanatophenyl-4-isocyanatophenyl sulfide, bis (4-isocyanatophenyl)sulfide and bis(4-isocyanatomethylphenyl)sulfide;
aromatic disulfide based polyisocyanate compounds such as bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide and bis(4-methoxy-3-isocyanatophenyl)disulfide;
sulfur-containing alicyclic polyisocyanate compounds such as 2,5-diisocyanato tetrahydrothiophene, 2,5-diisocyanato methyl tetrahydrothiophene, 3,4-diisocyanato methyl tetrahydrothiophene, 2,5-diisocyanato-1,4-dithiane, 2,5-diisocyanatomethyl-1,4-dithiane, 4,5-diisocyanato-1,3-dithiolane, 4,5-bis(isocyanatomethyl)-l,3-dithiolane and 4,5-diisocyanatomethyl-2-methyl-1,3-dithiolane;
aliphatic polyisothiocyanate compounds such as 1,2-diisothiocyanatoethane and 1,6-diisothiocyanatohexane; alicyclic polyisothiocyanate compounds such as cyclohexane diisothiocyanate; and aromatic polyisothiocyanate compounds such as 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4,4'-methylenebis(phenylisothiocyanate), 4,4'-methylenebis(2-methylphenylisothiocyanate), 4,4'-methylenebis(3-methylphenylisothiocyanate), 4,4'-diisothiocyanatobenzophenone, 4,4'-diisothiocyanato-3,3'-dimethylbenzophenone, and bis (4-isothiocyanatophenyl)ether.

Further examples thereof include carbonyl isothiocyanate compounds such as 1,3-benzenedicarbonyl diisothiocyanate, 1,4-benzenedicarbonyl diisothiocyanate, and (2,2-pyridine)-4,4-dicarbonyl diisothiocyanate; sulfur-containing aliphatic isothiocyanate compounds such as thiobis(3-isothiocyanatopropane), thiobis (2-isothiocyanatoethane) and dithiobis(2-isothiocyanatoethane);
sulfur-containing aromatic polyisothiocyanate compounds such as 1-isothiocyanato-4-[(2-isothiocyanato)sulfonyl]benzene, thiobis(4-isothiocyanatobenzene), sulfonyl(4-isothiocyanatobenzene) and dithiobis(4-isothiocyanatobenzene); sulfur-containing alicyclic compounds such as 2,5-diisothiocyanatothiophene and 2,5-diisothiocyanato-1,4-dithiane; and
compounds having an isocyanate group and an isothiocyanate group such as 1-isocyanato-6-isothiocyanatohexane, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanatophenyl-4-isothiocyanatophenyl sulfide and 2-isocyanatcethyl-2-isothiocyanatoethyl disulfide. But the present invention is not restricted to using these exemplified compounds.

Furthermore, these compounds may be substituted with halogen, such as chlorine or bromine, alkyl, alkoxy, or nitro. Moreover, these compounds may be modified with polyalcohol (prepolymer type), carbodiimide, urea, or biuret. Dimer or trimer reaction products of these compounds may also be used. These compounds may be used singly or in combination of two or more kinds.

The proportion of the pentaerythritol mercaptocarboxylic acid ester and the polyiso (thio) cyanate compound used is usually in the range of 0.3 to 2.0 and preferably in the range of 0.7 to 2.0 as the ratio of SH group to NCO group.

For purposes of improvement of general properties, usability and polymerization responsiveness required for the polyurethane based resin to be described later, one or two or more kinds of material (s) other than urethane-forming raw materials, such as active hydrogen compounds represented by amine, epoxy compounds, olefin compounds, carbonate compounds, ester compounds, metals, metal oxides, organic metal compounds and inorganic substances, may be added to the polymerizable composition forming a urethane resin, in addition to the foregoing ester and iso(thio)cyanate compound.

Furthermore, various substances may be added in the same manner as in a known molding method depending on the purposes. Examples of the substance include a chain extension agent, a crosslinking agent, a light stabilizer, an ultraviolet absorber, an anti-oxidant, an oil soluble dye, a filler, a mold release agent and a blueing agent. A known reaction catalyst used in the production of thiocarbamic acid S-alkyl ester or polyurethane may be properly added for the purpose of adjusting to the desired reaction rate. The polyurethane based resin
is usually obtained by a casting polymerization.

Specifically, the pentaerythritol mercaptocarboxylic acid ester and the polyiso(thio)cyanate compound are mixed. The resulting mixture is degassed by an adequate method, if necessary. Subsequently, the mixture is injected into a mold and usually heated from a low temperature to a high temperature slowly for polymerization.

The thus-obtained polyurethane based resin usually has properties of high refractive index and low dispersion, excellent heat resistance, excellent durability, light weight and excellent impact resistance. Furthermore, generation of whitening is suppressed by the effect of the present invention, and the resin is suitable for use in an optical element material of spectacle lenses, camera lenses and the like.

Furthermore, an optical element can comprise such a resin. As such an optical element, for example, a lens may be cited. Such a lens is obtained by a usual casting polymerization.

Further, the polyurethane based lens produced according to an embodiment of the present invention may undergo physical or chemical processes for purposes of improvement of anti-reflection, high hardness grant, improvement of wear resistance, improvement of chemical resistance, anti-fogging property grant or fashionability grant, such processes include surface polishing, antistatic process, hard coat process, non-reflective coat process, dyeing process and photochromic process.

### EXAMPLES

The present invention is illustrated in detail below with reference to Examples. Pentaerythritol in use was analyzed in the following manner. Furthermore, among performances of the obtained resin, refractive index, mold release properties, bubbles and transparency were evaluated in the following test method.

Content of bispentaerythritol: Pentaerythritol was dissolved in water and then the content was measured by high performance liquid chromatography.

Content of sodium: Pentaerythritol was dissolved in water and then the content was measured by high performance liquid chromatography.

Content of calcium pentaerythritol was incinerated, a solution was produced, and this was acidified with hydrochloric acid, and then diluted with pure water before being measured by using an inductively coupled plasma - atomic emission spectrometer.

Refractive index (ne) and Abbe number (ve): These were measured at 20 degree centigrade using a Pulfrich's refractometer.

Evaluation of mold release properties: It was evaluated by using a convex mold prepared with a glass mold having an outer diameter of 84 mm and a height of 17 mm, and a tape having an outer diameter of 84 mm and a height of 11 mm. Ten sets each thereof were introduced and the polymerization was completed, and then cooled down to room temperature. At that time when none of ten sets were broken or cracked, it was taken as AA. In other cases, it was taken as BB.

Evaluation of bubbles: A plastic lens was observed with a microscope at 100 magnifications. When there were bubbles inside, it was taken as BB. When there were no bubbles, it was taken as AA.

Transparency: A circular plate of ϕ75 mm having a thickness of 9 mm was prepared for measuring it with a gray-scale imaging device. When C brightness was not more than 50, it was taken as AA. When it was not less than 51, it was taken as BB.

### Example 1

### Synthesis of pentaerythritol(3-mercaptopropionic acid)ester

To a 1-liter 4-necked reaction flask equipped with a stirrer, a reflux condensing water separator, a nitrogen gas purge tube and a thermometer were added 143.0 weight parts (1.0 mole) of pentaerythritol having a purity of 95.0% containing 4.7 weight % of bispentaerythritol and 0.1 weight % of sodium portion (a metal compound was sodium alone), 4. 0 weight parts of p-toluene sulfonic acid monohydrate, 172.0 weight parts of toluene and 440.3 weight parts (4.15 mole) of 3-mercaptopropionic acid. The resulting material was reacted for 5 hours (internal temperature of 104 to 121 degree centigrade) while continuously removing by-produced water out of the system under heating reflux for cooling down to room temperature. The amount of water removed from the system was 99.0% based on theoretically generated water. The reaction solution was washed with a base and washed with water for removing toluene and trace moisture under heating reduced pressure, and then filtered to obtain 465.0 weight parts of a pentaerythritol-3-mercaptopropionic acid ester (hereinafter simply referred to as PEMP). APHA of the obtained PEMP was 10, while SHV was 7.81 eq/g.

### Production of plastic lens

87 weight parts of m-xylylene diisocyanate, 0.01 weight part of dibutyltin dichloride as a curing catalyst, 0.18 weight parts of Zelec UN (acid phosphate ester) and 0.10 weight part of BioSorb 583 (an ultraviolet absorber) were mixed and dissolved at 20 degree centigrade. 113 weight parts of the obtained PEMP was introduced and mixed to give a uniform mixed solution. The uniform solution was degassed at 600 Pa for 1 hour, and then filtered off using a 3-µm Teflon (registered trademark) filter, and then injected into a mold composed of glass molds and tapes. The mold was put into an oven, subjected to a temperature elevation from 10 degree centigrade to 120 degree centigrade slowly, and polymerized for 18 hours. When polymerization was terminated, the mold was taken out of the oven and released to obtain a resin. The obtained resin was further annealed at 120 degree centigrade for 3 hours. The obtained resin was excellent such that it was colorless and had high transparency. The refractive index (ne) was 1.600 and Abbe number (ve) was 35. The evaluation of mold release properties was AA, evaluation of bubbles was AA, and C brightness exhibiting the transparency was 20, corresponding to AA.

### Example 2

A pentaerythritol(3-mercaptopropionic acid)ester was synthesized in the same manner as in Example 1, except that pentaerythritol having a purity of 95.5% containing 4.0 weight % of bispentaerythritol and 0.02 weight % of calcium portion (a metal compound was calcium alone) instead of pentaerythritol used in Example 1. Using the obtained pentaerythritol(3-mercaptopropionic acid)ester, a plastic lens was produced and evaluated in the same manner as in Example 1. The evaluation results of the obtained plastic lens are shown in Table 1.

### Example 3

A pentaerythritol thioglycolic acid ester was synthesized in the same manner as in Example 1, except that 382.3 weight parts (4.15 mole) of thioglycolic acid was used instead of 3-mercaptopropionic acid. Using the obtained pentaerythritol thioglycolic acid ester, a plastic lens was produced and evaluated in the same manner as in Example 1. The evaluation results of the obtained plastic lens are shown in Table 1.

### Comparative Example A1

A pentaerythritol(3-mercaptopropionic acid)ester was synthesized in the same manner as in Example 1, except that pentaerythritol having a purity of 93.9% containing 5.5 weight % of bispentaerythritol and 0.2 weight % of sodium portion (a metal compound was sodium alone) instead of pentaerythritol used in Example 1. Using the obtained pentaerythritol(3-mercaptopropionic acid)ester, a plastic lens was produced and evaluated in the same manner as in Example 1. The evaluation results of the obtained plastic lens are shown in Table 1.

### Comparative Example 2

A pentaerythritol(3-mercaptopropionic acid)ester was synthesized in the same manner as in Example 1, except that pentaerythritol having a purity of 89.8% containing 1.0 weight % of bispentaerythritol and 3.1 weight % of sodium portion (a metal compound was sodium alone) instead of pentaerythritol used in Example 1. Using the obtained pentaerythritol(3-mercaptopropionic acid)ester, a plastic lens was produced and evaluated in the same manner as in Example 1. The evaluation results of the obtained plastic lens are shown in Table 1.

### Comparative Example 3

A pentaerythritol(3-mercaptopropionic acid)ester was synthesized in the same manner as in Example 1, except that pentaerythritol having a purity of 85.3% containing 5.5 weight % of bispentaerythritol and 3.1 weight % of sodium portion (a metal compound was sodium alone) instead of pentaerythritol used in Example 1. Using the obtained pentaerythritol(3-mercaptopropionic acid)ester, a plastic lens was produced and evaluated in the same manner as in Example 1. The evaluation results of the obtained plastic lens are shown in Table 1.

### Comparative Example 4

A pentaerythritol(3-mercaptopropionic acid)ester was synthesized in the same manner as in Example 1, except that pentaerythritol having a purity of 84.4% containing 5.5 weight % of bispentaerythritol and 3.1 weight % of calcium portion (a metal compound was calcium alone) instead of pentaerythritol used in Example 1. Using the obtained pentaerythritol(3-mercaptopropionic acid)ester, a plastic lens was produced and evaluated in the same manner as in Example 1. The evaluation results of the obtained plastic lens are shown in Table 1.

### Comparative Example 5

A pentaerythritol thioglycolic acid ester was synthesized in the same manner as in Example 3, except that pentaerythritol having a purity of 84.4% containing 5.5 weight % of bispentaerythritol and 3.1 weight % of calcium portion (a metal compound was calcium alone) instead of pentaerythritol used in Example 3. Using the obtained pentaerythritol thioglycolic acid ester, a plastic lens was produced and evaluated in the same manner as in Example 1. The evaluation results of the obtained plastic lens are shown in Table 1.

**[Table 1]**

| | Evaluation of plastic lens | | | | |
|---|---|---|---|---|---|
| | Refractive index (ne) | Abbe number (ve) | Mold release properties | Evaluation of bubbles | Transparency C brightness |
| Example 1 | 1.600 | 36 | AA | AA | AA |
| Example 2 | 1.600 | 36 | AA | AA | AA |
| Example 3 | 1.610 | 35 | AA | AA | AA |
| Comparative Example 1 | 1.600 | 36 | BB | BB | AA |
| Comparative Example 2 | 1.600 | 36 | AA | AA | BB |
| Comparative Example 3 | 1.600 | 36 | BB | BB | BB |
| Comparative Example 4 | 1.600 | 36 | BB | BB | BB |
| Comparative Example 5 | 1.610 | 35 | BB | BB | BB |

## Claims

1. A process for producing a pentaerythritol mercaptocarboxylic acid ester comprising reacting pentaerythritol of with a mercaptocarboxylic acid, wherein said pentaerythritol contains one or both of Na and Ca in a total of not more than 0.2% by weight, and contains not more than 5.0 wt% of bispentaerythritol.

2. The process for producing a pentaerythritol mercaptocarboxylic acid ester as set forth in claim 1, in which said pentaerythritol is subjected to a measure to reduce metal components.

3. The process for producing a pentaerythritol mercaptocarboxylic acid ester as set forth in claim 2, in which said measure to reduce metal components is an acid treatment.

4. The process for producing a pentaerythritol mercaptocarboxylic acid ester as set forth in claim 2, in which said measure to reduce metal components is a recrystallization method.

5. The process of any preceding claim including a further step of forming a polymerizable composition comprising the pentaerythritol mercaptocarboxylic acid ester and a polyiso(thio)cyanate compound.

6. The process of claim 5 including a step of forming a resin by curing the polymerizable composition set forth in claim 5.

7. The process of claim 6 which leads to an optical element comprising the resin.

8. The process of claim 6 which leads to a lens comprising the resin.

## Patentansprüche

1. Verfahren zur Herstellung eines Pentaerythritmercaptocarbonsäureesters, das das Umsetzen von Pentaerythrit mit einer Mercaptocarbonsäure umfasst, worin der Pentaerythrit eines oder beide von Na und Ca in einer Gesamtmenge von nicht mehr als 0,2 Gew.-% enthält und nicht mehr als 5,0 Gew.-% Bispentaerythrit enthält.

2. Verfahren zur Herstellung eines Pentaerythritmercaptocarbonsäureesters nach Anspruch 1, worin der Pentaerythrit einer Maßnahme zur Reduktion von Metallkomponenten unterzogen wird.

3. Verfahren zur Herstellung eines Pentaerythritmercaptocarbonsäureesters nach Anspruch 2, worin die Maßnahme zur Reduktion von Metallkomponenten eine Säurebehandlung ist.

4. Verfahren zur Herstellung eines Pentaerythritmercaptocarbonsäureesters nach Anspruch 2, worin die Maßnahme zur Reduktion von Metallkomponenten ein Umkristallisationsverfahren ist.

5. Verfahren nach einem der vorangegangenen Ansprüche, das einen weiteren Schritt des Ausbildens einer polymerisierbaren Zusammensetzung umfasst, die den Pentaerythritmercaptocarbonsäureester und eine Polyiso(thio)cyanatverbindung umfasst.

6. Verfahren nach Anspruch 5, das einen Schritt des Ausbildens eines Harzes durch Härten der in Anspruch 5 dargelegten polymerisierbaren Zusammensetzung umfasst.

7. Verfahren nach Anspruch 6, das ein das Harz umfassendes optisches Element ergibt.

8. Verfahren nach Anspruch 6, das eine das Harz umfassende Linse ergibt.

## Revendications

1. Procédé de production d'un ester de l'acide pentaérythritol mercaptocarboxylique comprenant la réaction du pentaérythritol avec un acide mercaptocarboxylique, où ledit pentaérythritol contient un ou les deux de Na et Ca en une quantité totale non supérieure à 0,2% en poids, et ne contient pas plus que 5,0% en poids de bispentaérythritol.

2. Procédé de production d'un ester de l'acide pentaérythritol mercaptocarboxylique selon la revendication 1, dans lequel ledit pentaérythritol est soumis à une mesure afin de réduire les composants métalliques.

3. Procédé de production d'un ester de l'acide pentaérythritol mercaptocarboxylique selon la revendication 2, dans lequel ladite mesure pour réduire les composants métalliques est un traitement à l'acide.

4. Procédé de production d'un ester de l'acide pentaérythritol mercaptocarboxylique selon la revendication 2, dans lequel ladite mesure pour réduire les composants métalliques est une méthode de recristallisation.

5. Procédé selon l'une quelconque des revendications précédentes, incluant une étape supplémentaire consistant à former une composition polymérisable comprenant l'ester de l'acide pentaérythritol mercaptocarboxylique et un composé polyiso(thio)cyanate.

6. Procédé selon la revendication 5, incluant une étape de formation d'une résine par le durcissement de la composition polymérisable selon la revendication 5.

7. Procédé selon la revendication 6, qui mène à un élément optique comprenant la résine.

8. Procédé selon la revendication 6, qui mène à une lentille comprenant la résine.
